# EUROPÄISCHE PATENTSCHRIFT

(11) **EP 3 374 892 B1**
(45) Veröffentlichungstag und Bekanntmachung des Hinweises auf die Patenterteilung: **13.09.2023**
(21) Anmeldenummer: 16793894.3
(22) Anmeldetag: 09.11.2016
(51) Int. Cl.: G16H 40/63, A61M 1/14

(54) **MEDIZINTECHNISCHES GERÄT MIT BEDIENERUNTERSTÜTZUNG UND VERFAHREN**
MEDICAL INSTRUMENT WITH USER ASSISTANCE AND METHOD
APPAREIL MÉDICAL À ASSISTANCE DE L'UTILISATEUR ET PROCÉDÉ

(30) Priorität: 11.11.2015 DE 102015014527
(43) Veröffentlichungstag der Anmeldung: 19.09.2018
(73) Patentinhaber: Fresenius Medical Care Deutschland GmbH, 61352 Bad Homburg (DE)
(72) Erfinder: WALDE, Jan-Willem, 35440 Linden (DE); KLEWINGHAUS, Jürgen, 61440 Oberursel (DE); RUDOLPH, Stefan, 99634 Werningshausen (DE); SCHROERS, Alexander, 60389 Frankfurt (DE); VERCH, Georg, 65191 Wiesbaden (DE); WITTZACK, Stefan, 37073 Göttingen (DE)
(74) Vertreter: Hitzelberger, Christoph Michael
(86) Internationale Anmeldenummer: PCT/EP2016/077151
(87) Internationale Veröffentlichungsnummer: WO 2017/081086

(56) Entgegenhaltungen:
- EP-A1- 0 904 788
- DE-A1-102011 121 668
- DE-A1-102012 020 945
- US-A1- 2009 113 335
- US-A1- 2014 230 071

## Beschreibung

### Technisches Gebiet

Die Erfindung liegt auf dem Gebiet der medizintechnischen Geräte, insbesondere Dialysegeräte, mit Bedienerschnittstelle.

### Hintergrund

Medizintechnische Geräte sind häufig mit Bedienerschnittstellen zur Ein- und Ausgabe von Information ausgerüstet. Übliche Bedienerschnittstellen sind Bildschirme wie LC-Bildschirme, TFT-Bildschirme oder OLED-Bildschirmen, die mit oder ohne Touchscreen-Funktionalität ausgeführt sein können. Neben der Anzeige von Informationen bieten Touchscreens-Displays auch eine komfortable und variable Eingabemöglichkeit für Informationen durch Fingerdruck des Bedieners auf die Touchscreenoberfläche und durch die flache Bauart auch eine leicht zu reinigende und damit hygienische Oberfläche.

Medizintechnische Geräte sind oftmals auch mit vielfältigen anderen Vorrichtungen zur Informationsausgabe und zur Informationseingabe ausgerüstet. So können akustische Signale von einem Lautsprecher ausgegeben werden. Akustische Eingaben können über Mikrofone durchgeführt werden. Auch optische Sensoren, wie Fingerabdruckscanner, oder Kameras zum Erfassen optischer Merkmale, können Teile von medizintechnischen Geräten sein.

Zur Bedienung des medizintechnischen Geräts verfügen diese häufig über spezielle grafische Bedienerführungen, die bevorzugt als grafische Bedieneroberfläche (GUI) ausgeführt sind. Solche bildschirmbasierten Bedienerführungen besitzen in der Regel eine vorgegebene Struktur mit Menu-Ansichten, unter denen bestimmte Funktionen, beispielsweise die Anzeige von Messwerten oder Eingabemöglichkeiten für einzustellende Parameter, wie beispielsweise Pumpraten oder Ähnliches, zusammengefasst sind.

Eine Menu-Ansicht ist hierbei definiert als die Anzeige von bestimmten Informationen zu einem Zeitpunkt. Hierbei umfassen die angezeigten Informationen auch Menu-Punkte zur Einstellung von Parametern. Beispielsweise kann ein Menu sowohl die Anzeige einer Pumprate einer bestimmten Pumpe des medizintechnischen Geräts umfassen, als auch die Anzeige eines Menu-Punkts, der vom Bediener aktiviert werden kann, wonach er die Pumprate eingeben bzw. verändern kann. Bei grafischen Bedieneroberflächen bei Geräten mit Touchscreen-Display erfolgt die Auswahl von Menu-Punkten in der Regel durch das Berühren der für diese Menu-Punkte vorgehaltenen Aktivierungsflächen (sog. Touchkeys).

Aus der EP 0 904 788 ist ein medizintechnisches Gerät bekannt, das eine symbolische Darstellung der betreffenden einzustellenden Geräteteile, wie Pumpen, auf einem Touchscreen-Display bereitstellt, wobei die Eingabe eines Parameters für dieses Geräteteil durch direktes Berühren des Symbols ermöglicht wird.

In der DE 10 2011 121 668 A1 wird zur komfortablen Programmierung des Ablaufs einer Behandlung mit einer medizintechnischen Vorrichtung vorgeschlagen, spezielle sogenannte Frontends vorzuhalten, in die ein Bediener den Ablauf der Behandlung eingibt, und wobei ein dem Frontend unterliegendes Interpreterprogramm die Eingaben des Bedieners in eine Phasenliste umwandelt, die der medizintechnischen Vorrichtung übermittelt wird, um sie entsprechend zu programmieren.

In der Regel umfasst die grafische Bedienerführung eine Vielzahl von Menu-Ansichten, um eine Vielzahl von Informationen, die das medizintechnische Gerät betreffen, und Eingabemöglichkeiten übersichtlich und thematisch geordnet darstellen zu können. Um bestimmte Menu-Ansichten aufzurufen, können sog. Sprungmarken vorgehalten werden, die vom Bediener aktiviert werden können, und beispielsweise von einem Haupt-Menu auf einzelne Unter-Menu-Ansichten verweisen. Auch Sprungmarken, die zwischen einzelnen Unter-Menu-Ansichten verweisen, sind bekannt.

Die grafische Bedienerführung des medizintechnischen Geräts kann für unterschiedliche Betriebsmodi des Geräts unterschiedlich komplex ausgeführt sein. Beispielsweise kann in einem sogenannten Service-Modus die grafische Bedienerführung eine besonders hohe Komplexität aufweisen, da in diesem Modus dem Bediener, beispielsweise einem Service-Techniker, der Zugriff auf besonders vielfältige Eingabemöglichkeiten und Informationen, die das medizintechnische Gerät betreffen, ermöglicht werden soll.

Andere Betriebsmodi, die angepasste grafische Bedienerführungen nach sich ziehen können, betreffen beispielsweise die Bedienung durch Anwendungsberater, Trainer oder Entwickler des medizintechnischen Geräts. Die jeweiligen grafischen Bedienerführungen sind an die Bedürfnisse des jeweiligen Bedieners angepasst und können jeweils eine unterschiedliche Komplexität aufweisen.

In solchen Betriebsmodi können zahlreiche Sonderfunktionen zur Bedienung, Einstellung, Wartung und Diagnose des Geräts in der jeweiligen grafischen Bedienerführung zur Verfügung stehen, die im normalen Betrieb des medizintechnischen Geräts während einer Behandlung eines Patienten nicht zur Verfügung stehen. Das Auffinden einer bestimmten Information oder einer Eingabemöglichkeit in der grafischen Bedienerführung gestaltet sich in der Regel mit zunehmender Komplexität der grafischen Bedienerführung zunehmend schwieriger.

So können bestimmte Informationen oder Eingabemöglichkeiten, je nachdem welche Menu-Ansicht gerade angezeigt wird, nicht unmittelbar durch eine Bedienereingabe anwählbar sein. In solchen Fällen ist die bestimmte Information oder Eingabemöglichkeit nicht Teil der aktuellen Menu- oder Unter-Menu-Ansicht. Der Bediener muss gegebenenfalls durch mehrfache Bedienereingabe zu einer Menu- bzw. Unter-Menu-Ansicht gelangen, die die bestimmte Information oder Eingabemöglichkeit beinhaltet. Die Anzahl der Bedienereingaben, die hierfür notwendig ist, steigt potentiell mit der Komplexität der grafischen Bedienerführung.

Der Bediener, der eine bestimmte Information sucht, oder eine bestimmte Eingabe am medizintechnischen Gerät tätigen möchte, muss entsprechend wissen, auf welcher Menu-Ansicht diese Information angezeigt wird, bzw. sich die Eingabemöglichkeit für die von ihm gewünschte Eingabe befindet.

Dies erfordert eine ausführliche Schulung bzw. lange Erfahrung im Umgang mit dem medizintechnischen Gerät.

### Zusammenfassung der Erfindung

Es ist daher die Aufgabe der vorliegenden Erfindung, die Bedienung eines medizintechnischen Geräts zu vereinfachen und sicherer zu gestalten.

Gelöst wird diese Aufgabe durch ein medizintechnisches Gerät nach Anspruch 1, sowie durch ein Verfahren nach Anspruch 10. Bevorzugte Ausführungsformen sind Gegenstand der Unteransprüche.

Demnach wird ein medizintechnisches Gerät und ein Verfahren für ein medizintechnisches Gerät vorgeschlagen, wobei das medizintechnische Gerät zumindest eine Eingabevorrichtung umfasst, die zur Eingabe von Informationen eingerichtet ist, die das medizintechnische Gerät betreffen, und wobei das medizintechnische Gerät zumindest eine Ausgabevorrichtung umfasst, die zur Ausgabe von Informationen eingerichtet ist, die das medizintechnische Gerät betreffen. Das medizintechnische Gerät umfasst zumindest ein Betriebsmittel, das beispielsweise als Aktor oder Sensor ausgeführt sein kann. Ein solches Betriebsmittel weist einen einstellbaren und/oder auslesbaren Betriebsparameter, wie beispielsweise eine Förderleistung einer Pumpe, und/oder eine von dem Betriebsmittel erzeugte und auslesbare Ausgangsgröße, wie beispielsweise einen Sensorwert eines Drucksensors auf.

Weiterhin umfasst das medizintechnische Gerät ein Steuergerät, welches datentechnisch zumindest mittelbar mit dem zumindest einen Betriebsmittel und der zumindest einen Eingabevorrichtung und der zumindest einen Ausgabevorrichtung verbunden ist, wobei das Steuergerät dazu konfiguriert ist, Informationen, die das medizintechnische Gerät betreffen, auszugeben und Eingabemöglichkeiten für die einstellbaren Betriebsparameter vorzuhalten.

Das Steuergerät hat datentechnischen Zugriff auf zumindest eine das medizintechnische Gerät betreffende Datei. Der datentechnische Zugriff kann über eine Netzwerkverbindung realisiert werden, eine weitere Ausführungsform sieht vor, die Datei in einem Datenspeicher des medizintechnischen Geräts abzuspeichern, auf den das Steuergerät datentechnischen Zugriff hat. Eine weitere Ausführungsform sieht vor, dass die Speicherung der zumindest einen Datei im Datenspeicher des medizintechnischen Geräts über eine Netzwerkverbindung erfolgt. Die zumindest eine das medizintechnische Gerät betreffende Datei ist in einer Ausführungsform ein abgespeichertes Dokument, beispielsweise eine Bedienungsanleitung mit Text und Grafikbestandteilen.

Das Steuergerät ist so konfiguriert, dass Textteile oder Grafiken, die ein Betriebsmittel betreffen, der zumindest einen das medizintechnische Gerät betreffenden Datei auswählbar sind.

Ein solcher ein Betriebsmittel betreffender Textteil kann beispielsweise ein einzelnes Wort sein, das ein Betriebsmittel bezeichnet, beispielsweise "Blutpumpe". Es können aber auch mehrere zusammenhörige Wörter, beispielsweise ein Satz, auswählbar sein, der sich auf ein Betriebsmittel bezieht, beispielsweise "Einstellung der Blutpumpenrate". Eine ein Betriebsmittel betreffende Grafik kann beispielsweise eine Abbildung des entsprechenden Betriebsmittel sein, oder auch eine symbolhafte Darstellung, beispielsweise ein Symbol innerhalb eines Hydraulikplans.

Nach der Auswahl eines solchen Textteils oder einer solchen Grafik kann zumindest ein Betriebsparameter des Betriebsmittels und/oder zumindest eine von dem betreffenden Betriebsmittel erzeugte und auslesbare Ausgangsgröße ausgegeben werden. Dies kann beispielsweise ein Wert eines Sensors, beispielsweise eines Blutdrucksensors sein.

Ebenfalls wird nach der Auswahl eines solchen Textteils oder einer solchen Grafik die Eingabe eines einstellbaren Betriebsparameters des betreffenden Betriebsmittels ermöglicht, wie beispielsweise für die einzustellende Förderrate einer Blutpumpe.

Erfindungsgemäß umfasst die zumindest eine das medizintechnische Gerät betreffende Datei ausschließlich textbasierte und/oder grafikbasierte Informationen, wie beispielsweise eine digital in der Datei abgespeicherte Bedienungsanleitung oder ein technisches Handbuch. Die Datei umfasst in diesem Fall keinen ausführbaren Programmcode, der zur Steuerung des medizintechnischen Geräts verwendet werden könnte.

Im Folgenden soll die vorliegende Erfindung am Beispiel eines Dialysegeräts als Beispiel einer Ausführungsform eines medizintechnischen Geräts dargelegt werden.

Dialysegeräte sind Blutbehandlungsgeräte, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin unter der Bezeichnung sleep.safe vertrieben werden, durchgeführt.

Dialysegeräte, als Beispiel für komplexe medizintechnische Geräte, haben umfangreiche Funktionen. Um diese Funktionen zu steuern, sind medizintechnische Geräte wie Dialysegeräte mit zumindest einem Steuergerät ausgerüstet. Diese können als CPU (central processing unit) oder Mikrokontroller ausgeführt sein, die von Softwareprogrammen programmiert werden. Im Lichte der Offenbarung der vorliegenden Erfindung ist es nicht wesentlich, ob die beschriebenen Verfahren von einem oder mehreren Steuergeräten durchgeführt werden. Demnach gilt auch eine Vielzahl von Steuergeräten als Steuergerät, solange sie datentechnisch miteinander verbunden sind. Die Softwareprogramme werden in der Regel in einer internen Speichervorrichtung vorgehalten. Weitere Speichervorrichtungen können zum Abspeichern sonstiger Informationen, wie Behandlungsdaten, vorgehalten werden.

Zur datentechnischen Verbindung mit externen Geräten können medizintechnische Geräte, wie Dialysegeräte, über Netzwerksschnittstellen verfügen.

Die Bedienung von Dialysegeräten erfolgt häufig über grafische Bedienerschnittstellen, die als Touchscreen-Display ausgeführt sein können. Hierbei werden grafische Bedienerführungen vorgehalten, die als grafische Bedieneroberfläche (GUI) ausgeführt sein kann. Solche bildschirmbasierenden Bedienerführungen besitzen in der Regel eine vorgegebene Struktur mit Menu-Ansichten, unter denen bestimmte Funktionen, beispielsweise die Anzeige von Messwerten oder Eingabemöglichkeiten für einzustellende Parameter, wie beispielsweise Pumpraten oder Ähnliches, zusammengefasst sind.

Eine als Touchscreen-Display ausgeführte grafische Bedienerschnittstelle ist eine Eingabe- und Ausgabevorrichtung im Sinne der vorliegenden Erfindung. Eine alternative Ausführungsform sieht vor, die grafische Bedienerschnittstelle auf getrennten Eingabe- und Ausgabevorrichtungen auszuführen, beispielsweise über ein Display und ein vom Display getrenntes Touchpad.

Eine weitere Möglichkeit zur Bedienung des medizintechnischen Geräts umfasst die akustische Ein- bzw. Ausgabe von Sprache. Der Fachmann bedient sich hierbei den bekannten Methoden der technischen Spracherzeugung bei der Ausgabe von Sprache durch das medizintechnische Gerät und der Spracherkennung bei der Eingabe von Sprache durch den Bediener. Eine Eingabevorrichtung im Sinne der vorliegenden Erfindung kann demnach eine Vorrichtung zur Eingabe akustischer Informationen sein und beispielsweise als Mikrofon ausgeführt werden. Eine Ausgabevorrichtung im Sinne der vorliegenden Erfindung kann demnach eine Vorrichtung zur Ausgabe akustischer Informationen sein und beispielsweise als Lautsprecher ausgeführt werden.

Das medizintechnische Gerät umfasst zumindest eine Eingabevorrichtung, die zur Eingabe von das medizintechnische Gerät betreffenden Informationen eingerichtet ist, und zumindest eine Ausgabevorrichtung, die zur Ausgabe von das medizintechnische Gerät betreffenden Informationen eingerichtet ist.

Es kann vorgesehen sein, dass die Eingabe und/oder die Ausgabe von das medizintechnische Gerät betreffenden Informationen ausschließlich mittels einer einzigen Eingabevorrichtung bzw. mittels einer einzigen Ausgabevorrichtung erfolgen. In einer alternativen Ausführungsform erfolgen die Eingabe und/oder die Ausgabe von das medizintechnische Gerät betreffenden Informationen mittels mehrerer Eingabevorrichtungen bzw. mittels mehrerer Ausgabevorrichtungen.

Um die Bedienung des Dialysegeräts einfacher und sicherer zu gestalten, schlägt die vorliegende Erfindung vor, Informationen, die das medizintechnische Gerät, hier das Dialysegerät betreffen, in digitaler Form als zumindest eine Datei vorzuhalten, auf die das Dialysegerät datentechnischen Zugriff hat, wobei Textteile oder Grafiken, die Betriebsmittel betreffen, der Datei auswählbar sind.

Die Auswählbarkeit eines solchen Textteils der zumindest einen das medizintechnische Gerät betreffenden Datei kann beispielsweisedurch eine Indexübersicht von Wörtern geschehen, die Betriebsmittel betreffen, wobei die Auswahl beispielsweise durch eine Bedienereingabe an einem Touchscreen-Display (Fingerdruck) auf ein Wort aus der Indexübersicht geschieht.

In einer alternativen Ausführungsform können auswählbare Textteile der zumindest einen das medizintechnische Gerät betreffenden Datei thematisch geordnet angezeigt werden, beispielsweise können Überschriften auswählbarer Textteile, die die Aufrüstung des Dialysegeräts betreffen, zusammen angezeigt werden. Eine derartige geordnete Anzeige von Überschriften auswählbarer Textteile kann beispielsweise durch die Auswahl des entsprechenden Oberbegriffs (hier also beispielsweise "Aufrüstung" oder "Aufrüstung des Dialysegeräts") in einer Indexübersicht von Wörtern wie weiter oben beschrieben erfolgen.

Eine weitere Ausführungsform sieht vor, dass Textteile oder Grafiken, die Betriebsmittel betreffen, ein Teil von einem auf der grafischen Bedienerschnittstelle angezeigten Ausschnitt der zumindest einen das medizintechnische Gerät betreffenden Datei sind, der seinerseits auswählbar sein kann. Derartige Ausschnitte der zumindest einen das medizintechnische Gerät betreffenden Datei können beispielsweise einzelne Seiten oder ganze Kapitel einer digital abgespeicherten Bedienungsanleitung oder eines technischen Handbuchs umfassen.

Weiterhin kann eine Suchfunktion implementiert sein, die über die Bedienerschnittstelle aufrufbar ist, wobei über die Suchfunktion die zumindest eine Datei nach Textstrings durchsuchbar ist. Ein Textstring kann ein einzelnes Wort oder ein Teil eines Worts sein oder mehrere zusammenhängende Wörter. Durch die Suchfunktion gefundene Textstrings können auswählbar sein, insbesondere dann, wenn der Textstring ein Betriebsmittel betrifft.

Die Ein- bzw. Ausgabe von Informationen, beispielsweise die Eingabe eines Textstrings, kann im Lichte der Offenbarung der vorliegenden Erfindung auch zumindest teilweise durch akustische Signale in Form von Sprache erfolgen. Beispielsweise kann der Bediener einen gesuchten Textstring auch aussprechen und so eine Spracheingabe durchführen. Das medizintechnische Gerät ist in diesem Fall mit einem Mikrofon ausgestattet und so konfiguriert, dass die Spracheingabe unter Verwendung von dem Fachmann bekannten Spracherkennungsverfahren digitalisiert wird und so dem Steuergerät als eingegebener Textstring zugeführt wird. In gleicher Weise kann die Informationsausgabe durch das medizintechnische Gerät auch zumindest teilweise über eine Sprachausgabe erfolgen.

Medizintechnische Geräte mit Sprachausgabe und Sprachsteuerung zumindest einzelner Funktion des medizintechnischen Gerät sind dem Fachmann aus dem Stand der Technik, beispielsweise aus der DE 109 624 988A1, der US 5,970,457 oder aus der DE 108 26 539 A1 bekannt. Die im Folgenden beschriebenen Ausführungsformen der vorliegenden Erfindung können für die Ein- und Ausgabe von Information zumindest teilweise auf gesprochene Sprache zurückgreifen.

Ein Betriebsmittel im Sinne der vorliegenden Erfindung ist ein Teil des medizintechnischen Geräts, das zumindest mittelbar mit einem Steuergerät des medizintechnischen Geräts signaltechnisch verbunden ist. Solche Betriebsmittel sind beispielsweise Aktoren, wie Motoren, Pumpen und Ventile, sowie Sensoren, wie Temperatursensoren oder Blutdrucksensoren. Betriebsmittel können auch Zubehörteile oder Anbauteile des medizintechnischen Geräts sein, die zumindest mittelbar mit einem Steuergerät des medizintechnischen Geräts signaltechnisch verbunden sind.

Die Betriebsmittel sind im Hinblick auf einen Betriebsparameter einstellbar, beispielsweise kann ein Ventil angesteuert werden, zu öffnen oder zu schließen, oder eine Pumpe so angesteuert werden, dass sich eine bestimmte Pumprate einstellt.

Betriebsmittel können überdies selbst Ausgangsgrößen erzeugen. Dies ist insbesondere bei Sensoren der Fall. Die Ausgangsgröße eines Blutdrucksensors, der am extrakorporalen Blutkreislauf einer Dialysemaschine angebracht ist, ist beispielsweise der an dieser Stelle des extrakorporalen Blutkreislaufs gerade herrschende Flüssigkeitsdruck des Blutes bzw. eine dafür charakteristische Ausgangsgröße, beispielsweise eine charakteristische elektrische Spannung oder ein charakteristischer elektrischer Widerstand.

Ein Beispiel für ein Betriebsmittel, das sowohl einstellbar ist, als auch selbst Ausgangsgrößen erzeugt, ist eine Schlauchrollenpumpe mit einem Sensor zur Erkennung der Umdrehungsgeschwindigkeit des Rotorarms. Der Motor der Schlauchrollenpumpe kann über das Steuergerät angesteuert werden, um eine bestimmte Umdrehungsgeschwindigkeit des Rotorarms einzunehmen, um somit abhängig vom eingelegten Schlauch eine bestimmte Pumprate zu erzeugen.

Der Sensor zur Erkennung der Umdrehungsgeschwindigkeit des Rotorarms erkennt die tatsächliche Umdrehungsgeschwindigkeit des Rotorarms. Diese Information kann beispielsweise dazu verwendet werden, um eine einzustellende Umdrehungsgeschwindigkeit auch unter erschwerter Last beizubehalten, in dem die Ist-Größe an dias Steuergerät zurückgekoppelt wird, die daraufhin die Ansteuerung des Pumpenmotors anpasst.

Betriebsmittel im Sinne der Erfindung sind also durch Betriebsparameter, die einstellbar und/oder auslesbar sind, wie beispielsweise die Umdrehungsgeschwindigkeit des Rotorarms einer Schlauchrollenpumpe und/oder durch auslesbare Ausgangsgrößen, wie der gemessene Blutdruck eines Blutdrucksensors, gekennzeichnet.

Ein Textteil der Datei, der Betriebsmittel betrifft, kann im einfachsten Fall eine Bezeichnung des Betriebsmittels, beispielsweise "Blutpumpe" sein. Weiterhin können auch mehrere zusammenhängende Wörter, beispielsweise "Einstellung der Blutpumpe" ein Textteil des angezeigten Teils der Datei sein, der Betriebsmittel betrifft.

Eine Grafik innerhalb der Datei, die ein Betriebsmittel betrifft, kann beispielsweise eine Abbildung des entsprechenden Betriebsmittel sein, oder auch eine symbolhafte Darstellung, beispielsweise ein Symbol innerhalb eines Hydraulikplans. Um dem Bediener das Auffinden des betreffenden Betriebsmittels innerhalb eines solchen Hydraulikplans, oder generell in technischen Plänen zu erleichtern, kann dieses grafisch hervorgehoben dargestellt werden, beispielsweise durch eine Umrahmung, eine bestimmte Farbgebung, durch ein blinkende Darstellung oder durch Pfeile, die auf das Bauteil zeigen.

Der Bediener eines medizintechnischen Geräts ist in der täglichen Arbeit daran interessiert, einfach und schnell die Betriebsparameter des medizintechnischen Geräts in Erfahrung zu bekommen, oder sie einzustellen. Durch die im Einklang mit der vorliegenden Lehre vorgeschlagene Auswählbarkeit von Textteilen oder Grafiken, die ein Betriebsmittel betreffen, wonach das ausgewählte Betriebsmittel eingestellt und eine von dem ausgewählten Betriebsmittel erzeugte Ausgangsgröße angezeigt werden kann, wird eine einfache und schnelle und somit auch sichere Bedienbarkeit des medizintechnischen Geräts gewährleistet.

So kann ein Bediener des medizintechnischen Geräts beispielsweise nach dem Textstring "Blutpumpe" durch Benutzen der Suchfunktion innerhalb der zumindest einen zugänglichen Datei suchen, wenn er die Blutpumpenrate überprüfen und/oder einstellen möchte. Die Suchfunktion bietet in einer Ausführungsform hiernach entsprechende Ausschnitte der Datei zur Auswahl an, die diesen Textstring beinhalten. Innerhalb eines solchen Ausschnitts der Datei kann der Textstring "Blutpumpe" als Textteil, der ein Betriebsmittel betrifft, eines angezeigten Ausschnitts der Dateiselbst wiederum auswählbar sein. Ein angezeigter Ausschnitt der Datei kann beispielsweise eine Seite oder ein Kapitel einer Bedienungsanleitung oder eines technischen Handbuchs sein.

Wählt der Bediener einen solchen Textteil, der ein Betriebsmittel betrifft, innerhalb des ausgewählten Ausschnitts der Datei aus, sieht die vorliegende Lehre vor, dass Betriebsparameter des betreffenden Betriebsmittels, also beispielsweise die eingestellte Umdrehungsgeschwindigkeit der als Schlauchrollenpumpe ausgeführten Blutpumpe, und/oder zumindest eine von dem betreffenden Betriebsmittel erzeugte und auslesbare Ausgangsgröße, beispielsweise die vom Sensor zur Erkennung der Umdrehungsgeschwindigkeit des Rotorarms der Blutpumpe erkannten tatsächlichen Umdrehungsgeschwindigkeit, auf der grafischen Bedienerschnittstelle angezeigt werden.

Weiterhin kann zusätzlich zu der beschriebenen Anzeige des Betriebsparameters nach Auswahl eines Textteils oder einer Grafik innerhalb des angezeigten Teils der Datei, die ein Betriebsmittel betreffen auch eine Eingabemöglichkeit für die einstellbaren Betriebsparameter des betreffenden Betriebsmittels, also beispielsweise ein Fenster, dass die Eingabe einer Umdrehungsgeschwindigkeit für die Blutpumpe ermöglicht, auf der grafischen Bedienerschnittstelle angezeigt werden.

In einer alternativen Ausführungsform kann die Suchfunktion auch Textteile, die ein Betriebsmittel betreffen und durch den eingegebenen Textstring bezeichnet werden (beispielsweise "Blutpumpe"), zur Auswahl direkt anbieten. Eine Anzeige beispielsweise einer Seite oder eines Kapitel einer Bedienungsanleitung oder eines technischen Handbuchs, die als Ausschnitt der Dateieinen Textstring enthalten, der das entsprechende Betriebsmittel betrifft, ist somit nicht zwingend erforderlich. Das Betriebsmittel kann dementsprechend direkt angewählt werden.

Es ist vorgesehen, dass die Suchfunktion innerhalb der strukturierten Bedienerführung möglichst unmittelbar aufrufbar ist. Dies kann beispielsweise dadurch realisiert werden, dass eine entsprechende Berührfläche (sog. Touch-Key) auf allen, oder einer Vielzahl von Menu-Ansichten zur Verfügung steht. Somit kann ein Bediener eines medizintechnischen Geräts einfach und schnell zu bestimmten Informationen bzw. Eingabemöglichkeiten gelangen, weitgehend unabhängig davon, welche Menu-Ansicht gerade angezeigt wird.

### Kurzbeschreibung der Zeichnungen

Weitere Einzelheiten und Vorteile der Erfindung werden anhand von in den Zeichnungen dargestellten Ausführungsbeispielen näher beschrieben. Es zeigen:
Die Figur 1 ein beispielhaft als Hämodialysegerät ausgeführtes medizintechnisches Gerät nach dem Stand der Technik;
die Figur 2 beispielhafte Bildschirminhalte einer Bedienerschnittstelle eines erfindungsgemäßen medizintechnischen Geräts;
die Figur 3 einen weitere beispielhafte Bildschirminhalte einer Bedienerschnittstelle eines erfindungsgemäßen medizintechnischen Geräts;
die Figur 4 den prinzipiellen Aufbau eines erfindungsgemäßen medizintechnischen Geräts und
die Figur 5 ein Ablaufdiagramm eines erfindungsgemäßen Verfahrens.

### Detaillierte Beschreibung der Figuren

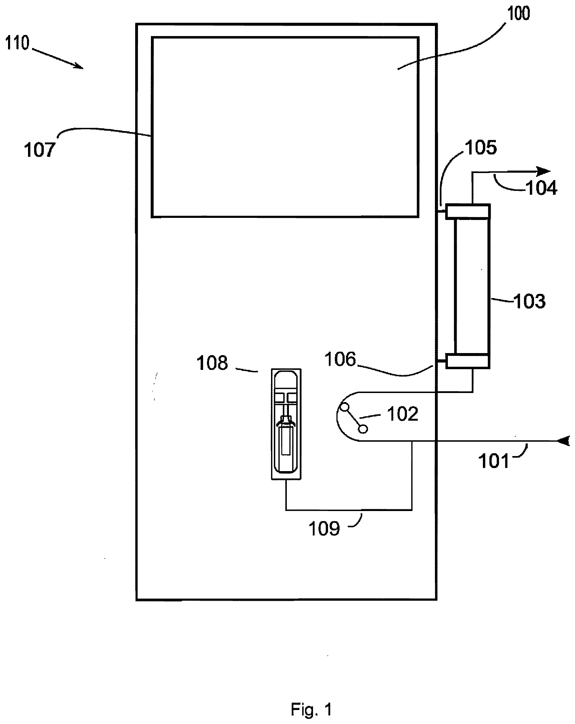

In Figur 1 ist ein als Hämodialysegerät als Beispiel für ein medizintechnisches Gerät schematisch dargestellt. Das Hämodialysegerät 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen. Um das Gerinnen des Blutes im extrakorporalen Blutkreislauf zu verhindern, ist vorgesehen, dem Blut ein gerinnungshemmendes Mittel zuzusetzen. Dies ist in Figur 1 mit der Heparinpumpe 108 gezeigt, die als Spritzenpumpe ausgeführt ist und über die Leitung 109 Heparin als Antikoagulanz in die arterielle Blutleitung 101 fördert.

Das Hämodialysegerät 110 ist mit einem Touchscreen-Display 100 ausgerüstet. Das Touchscreen-Display 100 dient als grafische Bedienerschnittstelle, die zur Ein-und Ausgabe von Informationen eingerichtet ist. Auf diesem Touchscreen-Display 100 wird zur Bedienung des Dialysegeräts 110 eine grafische Bedienerführung, beispielsweise eine grafische Bedieneroberfläche (GUI) ausgeführt.

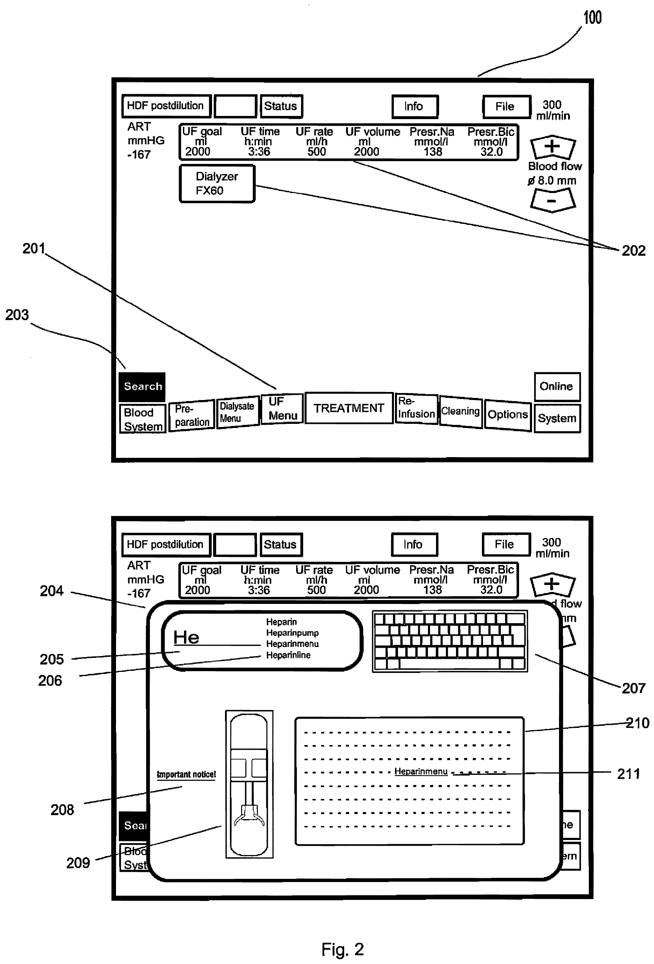

In Figur 2 sind erfindungsgemäße Bildschirminhalte der grafischen Bedieneroberfläche beispielhaft dargestellt. Die dargestellte Bedieneroberfläche hat beispielsweise die Struktur eines Menus, wobei von jeweils einer aktuellen Ansicht der Bedieneroberfläche zu weiteren Ansichten, beispielsweise Untermenus, gewechselt werden kann.

In der oberen Bildschirmansicht der Figur 2 ist eine typische Bedieneroberfläche eines Dialysegeräts dargestellt. Angezeigt werden Bereiche, die Informationen über die gerade laufende Behandlung darstellen, wie die Bereiche 202, die Auskunft über den verwendeten Dialysefilter (FX60), sowie über aktuelle Messwerte oder Parameter zeigen, wie beispielsweise die Gesamtmenge des zu entziehenden Plasmawassers (in Figur 2 2000ml) oder die Ultrafiltrationsrate (in Figur 2 500ml pro Stunde).

Weiterhin können über Berührflächen bzw. Touchkeys, wie in Figur 2 beispielsweise mit 201 gekennzeichnet, Eingaben in die Bedieneroberfläche getätigt werden, um beispielsweise zu anderen Ansichten bzw. Menus zu gelangen.

Im Einklang mit der vorliegenden Lehre verfügt die Bedieneroberfläche über eine Suchfunktion, die in Figur 2 durch Berühren der Berührfläche 203 aktiviert werden kann.

In der unteren Bildschirmansicht der Figur 2 ist die Situation beispielhaft nach Aktivieren der Suchfunktion dargestellt. Ein Fenster 204, dass die darunter liegende Ansicht teilweise verdeckt, erscheint. Der Bediener kann über eine virtuelle Tastatur 207 Textstrings in die Bedienerschnittstelle eingeben. Die Eingaben werden an der Stelle 205 angezeigt.

Ein eingegebener Textstring kann innerhalb von Dateien gesucht werden, auf die das medizintechnische Gerät datentechnischen Zugriff hat.

Es kann vorgesehen sein, dass schon während der Eingabe des Textstrings eine Auswahl an Textstrings angezeigt wird, die hinsichtlich ihrer Schreibweise oder hinsichtlich ihrer Aussprache zumindest teilweise mit dem eingegebene Begriff übereinstimmen (Figur 2, 206). Ein aus den solchermaßen angezeigten Textstrings ausgewählter Textstring wird anschließend als der in die Suchfunktion eingegebene Textstring behandelt.

Es kann weiterhin vorgesehen sein, dass eine automatische Korrektur von Rechtschreibefehlern bei der Eingabe vorgenommen wird. Vertauscht der Bediener beispielsweise die Buchstabenreihenfolge an einer Stelle eines Textstrings versehentlich, kann vorgesehen sein, dass dennoch eine Auswahl an Textstrings angezeigt wird, die hinsichtlich ihrer Schreibweise oder hinsichtlich ihrer Aussprache zumindest teilweise mit dem korrekt geschriebenen Begriff übereinstimmen. Der Fachmann bedient sich bei der Realisierung derartiger Ausführungsformen beispielsweise bei den Verfahren der Autokorrektur, die aus Textverarbeitungsprogrammen bekannt sind.

Die solchermaßen in den gesuchten Dateien gefundenen Textstrings werden auf der Bedienerschnittstelle angezeigt.

Der Bediener kann aus den angezeigten Textstrings 206 beispielsweise durch Berühren einen bestimmten Textstring auswählen. Daraufhin werden ein oder mehrere Ausschnitte der Datei, die den ausgewählten Begriff beinhalten, angezeigt. In Figur 2 wurde beispielsweise der Textstring "Heperinpumpe" ausgewählt, woraufhin in einem Bereich 210 ein Ausschnitt der Datei, symbolisiert durch unterbrochene waagerechte Linien, die den Begriff "Heparinmenue" beeinhaltet (211, Figur 2 im Klartext und unterstrichen), angezeigt.

Der Text kann beispielsweise einer in einer Datei gespeicherten Betriebsanleitung entnommen sein. Der Text kann aber auch aus einer beliebigen anderen Datei entnommen werden, die ausschließlich textbasierte und/oder grafikbasierte Informationen umfasst und keinen ausführbaren Programmcode, der zur Steuerung des medizintechnischen Geräts verwendet werden könnte, beispielsweise einem technischen Handbuch. Es kann vorgesehen sein, dass der Bediener zwischen mehreren Ausschnitten der Datei oder mehrerer Dateien, die den ausgewählten Begriff beinhalten, wählen kann. Dies kann beispielsweise über eine Auflistung von kurzen Textstücken aus verschiedenen Dokumenten erfolgen, aus denen der Bediener auswählen kann. Eine andere Ausführungsform sieht vor, dass der Bediener durch einen Steuerbefehl, beispielsweise einer Bedienereingabe an der Bedienerschnittstelle, mehrere Ausschnitte der Datei oder mehrerer Dateien, die den ausgewählten Begriff beinhalten, nacheinander aufrufen kann.

Es ist für die vorliegende Lehre unerheblich, wo die zumindest eine Datei, die das medizintechnische Gerät betrifft, gespeichert ist. Wesentlich ist, dass das Steuergerät darauf Zugriff hat. So kann die zumindest eine Datei in einem internen Speicher des medizintechnischen Geräts abgelegt sein. Eine weitere Ausführungsform sieht vor, dass die zumindest eine Datei in einem anderen Gerät, beispielsweise in einem Zentralrechner, oder in einem Zentralspeicher, beispielsweise einem sog. Cloud-Speicher, abgelegt sind, und das medizintechnische Gerät über eine Netzwerkverbindung auf die zumindest eine Datei zugreifen kann.

Eine externe Speicherung in externen Geräten ermöglicht den Zugriff mehrerer medizintechnischer Geräte auf die zumindest eine Datei über Netzwerkverbindungen. Wird eine solchermaßen abgespeicherte Datei also geändert, entfaltet das automatisch auf alle mit dem externen Gerät oder den externen Geräten über eine Netzwerkverbindung verbundenen medizintechnischen Geräte Wirkung. Somit wird die Aktualisierung der Datei für viele medizintechnische Geräte gleichzeitig ermöglicht.

Weiterhin kann vorgesehen sein, dass die zumindest eine Datei auch Bilder oder Zeichnungen enthält, die einen Bezug auf den ausgewählten Begriff haben. In Figur 2 ist beispielsweise die Abbildung 209 einer Heparinpumpe angezeigt.

Ebenfalls können Nachrichten, oder Hinweise auf anzuzeigende Nachrichten, die einen Bezug auf den ausgewählten Textteil oder den ausgewählten Ausschnitt der Datei haben, angezeigt werden (208, Fig. 2). Dies ermöglicht es, dem Bediener zusätzliche, in den normalen Dokumenten wie Betriebsanleitungen oder technischen Handbüchern nicht enthaltene Informationen zugänglich zu machen. Die Anzeige der Nachricht erfolgt beispielsweise durch Auswählen des Hinweises auf eine Nachricht (208) durch Berühren. Derartige Nachrichten können beispielsweise aktuelle Erkenntnisse enthalten, die durch die Bedienung des medizintechnischen Geräts gewonnen wurden und dem Bediener zur Kenntnis gegeben werden sollen.

Solche Nachrichten können im Einklang mit der vorliegenden Lehre ebenfalls in Dateien gespeichert sein, auf die das medizintechnische Gerät datentechnischen Zugriff hat.

Generell kann die Anzeige von Text, Bildern oder Zeichnungen im Einklang mit der vorliegenden Lehre auch durch einen Internetbrowser erfolgen, insbesondere wenn durch die Auswahl von angezeigten Begriffen ein sogenannter Hyperlink auf Webseiten des Internet aktiviert wird.

Der Bediener kann Textteile oder Grafiken in dem angezeigten Teil der Datei auswählen. In Figur 2 ist die Auswählbarkeit des Begriffes 211 durch dessen Unterstreichung gekennzeichnet. Beliebige andere Kennzeichen, beispielsweise auch die Farbe der Begriffe, können verwendet werden, um die Auswählbarkeit anzuzeigen.

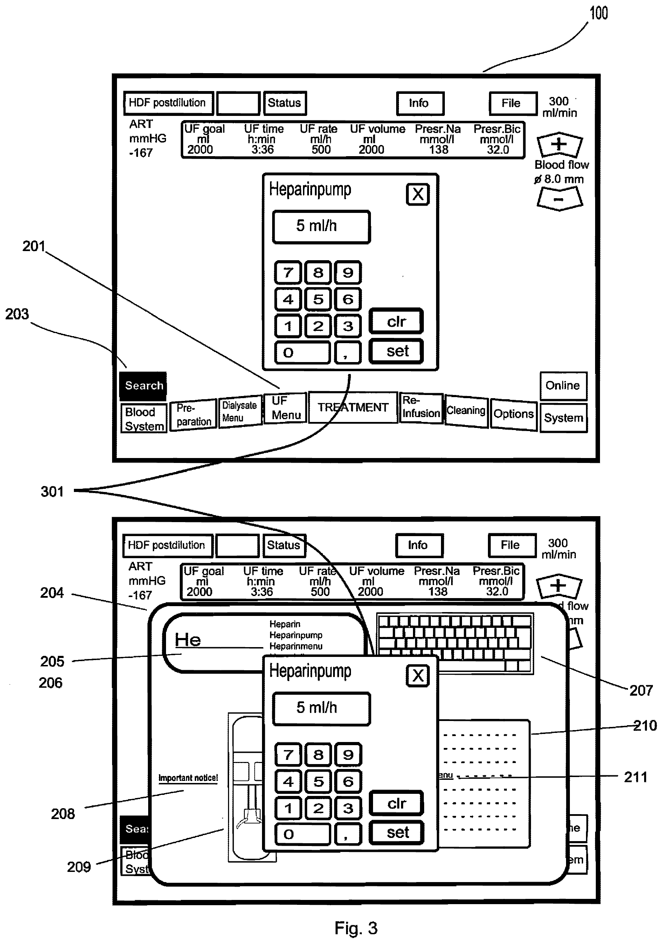

Durch Auswahl eines Textteils oder einer Grafik in der angezeigten Passage erhält der Bediener weitere Möglichkeiten, um Informationen abzurufen oder der Eingabe von Information in die Bedienerschnittstelle, wie in Figur 3 gezeigt.

In Figur 3 ist in der oberen Bildschirmansicht in einer erste Ausführungsform dargestellt, dass nach Anwahl des Begriffs 211 die Suchfunktion beendet wird und das Menu für die Anzeige und Einstellung der Heparinpumpenfunktion aufgerufen wird.

In einer weiteren Ausführungsform, dargestellt in der Figur 3 in der unteren Bildschirmansicht, wird die Suchfunktion nicht beendet. Das Menu für die Anzeige und Einstellung der Heparinpumpenfunktion überdeckt hier Teile des Fensters 204.

In einer weiteren Ausführungsform, die nicht in den Figuren 2 und 3 dargestellt ist, wird nach Eingabe eines Textstrings in die Suchfunktion direkt durch die Auswahl eines durch die Suchfunktion angezeigten und auswählbaren Textstrings 206 das Menu 301 für die Anzeige und Einstellung eines Parameters eines Betriebsmittels aufgerufen, sofern der ausgewählte Textstring dieses Betriebsmittel betrifft.

Die Anzeige eines Ausschnitts der Datei, der den ausgewählten Textstring betrifft (210) und die Auswahl eines dort angezeigten Textteils 211, der das Betriebsmittel betrifft, ist in dieser Ausführungsform nicht zwingend notwendig.

In einer alternativen Ausführungsform kann parallel zum Aufrufen des Menus 301 für die Anzeige und Einstellung eines Parameters eines Betriebsmittels auch die Anzeige eines Ausschnitts der Datei, der den ausgewählten Textstring betrifft, erfolgen. Dies erfolgt im Unterschied zur Darstellung in Figur 3 unten bevorzugt derart, dass das Menu 301 für die Anzeige und Einstellung eines Parameters eines Betriebsmittels und die Anzeige des Ausschnitts der Datei, der den ausgewählten Textstring betrifft, im Fenster 210 nicht überlappend auf der grafischen Bedieneroberfläche positioniert werden. So ist die vollständige Lesbarkeit der Information im angezeigten Ausschnitt der Datei, der den ausgewählten Textstring betrifft, im Fenster 210 bei gleichzeitiger Bedienbarkeit des Menus 301 für die Anzeige und Einstellung eines Parameters eines Betriebsmittels sicher gestellt.

In allen Fällen hat der Bediener die Möglichkeit, den Betriebsparameter des Betriebsmittels, das für ihn von Interesse ist (in den Figuren 2 und 3 Heparinfluss der Heparinpumpe) schnell und unkompliziert einzusehen und einzustellen.

Somit muss der Bediener nicht wissen, an welcher Stelle der Menustruktur der Bedieneroberfläche Zugriff auf diesen Betriebsparameter möglich ist. Durch die Suchfunktion, die in einer Ausführungsform von beliebigen Stellen innerhalb der Menustruktur der Bedieneroberfläche aufrufbar ist, kann nach dem Betriebsmittel gesucht werden und das Menu für die Anzeige und Einstellung der Betriebsparameter des gesuchten Betriebsmittels durch Auswahl des Betriebsmittels, bzw. eines Textteils oder einer Grafik innerhalb des angezeigten Ausschnitts der Datei, die sich auf das Betriebsmittel beziehen, aufgerufen werden.

Es kann weiterhin vorgesehen sein, dass die Anzeige von Information oder die Bereitstellung von Eingabemöglichkeiten für Parameter von einer zuvor dem medizintechnischen Gerät bekannt gemachten Autorisation des Bedieners abhängt. So kann es vorgesehen sein, dass bestimmte Personen Information einsehen, aber Betriebsparameter nicht verändern dürfen. Beispielsweise kann es vorgesehen sein, dass Pflegekräfte zwar die Information über die Blutpumpenrate einsehen dürfen, aber keine Möglichkeit haben, diese neu einzustellen, wohingegen Ärzte diese Möglichkeit haben.

Somit wird sichergestellt, dass nur autorisierte Personen bestimmte Einstellungen an dem medizintechnischen Gerät vornehmen dürfen. Auch kann die Informationsanzeige von der Autorisation des Bedieners abhängig gemacht werden. Beispielsweise kann Ärzten eine umfangreichere Auswahl an Textstrings (206) angeboten werden als Pflegepersonal.

Zur Einlesen der Autorisation des Bedieners kann das medizintechnische Gerät über eine Autorisierungsvorrichtung verfügen, die beliebig ausgeführt sein kann. Beispielsweise kann das medizintechnische Gerät mit einem Kartenlesegerät ausgestattet sein. Ein solches Kartenlesegerät dient zum Einlesen der Informationen, die auf einer sog. elektronischen SmartCard gespeichert ist und Angaben zur Person, beispielsweise den Autorisierungsgrad, umfassen kann. Das bedienende Personal kann sich somit durch das Einlesen der mitgeführten SmartCard an dem medizintechnischen Gerät autorisieren.

Durch eine solche Autorisierung kann das medizintechnische Gerät im Lichte der vorliegenden Erfindung auch in verschiedene Betriebsmodi überführt werden. Beispielsweise kann ein Service-Techniker durch seine Autorisierung am medizintechnischen Gerät das medizintechnische Gerät in einen Service-Modus überführen, in dem eine speziell für diesen Betriebsmodus angepasste grafische Bedienerführung Zugriff auf vielfältige Einstellmöglichkeiten und Informationen zur Diagnose, Einstellung und Wartung des medizintechnischen Geräts erlaubt. Andere Betriebsmodi, die angepasste grafische Bedienerführungen nach sich ziehen, betreffen beispielsweise die Bedienung durch Anwendungsberater, Trainer oder Entwickler des medizintechnischen Geräts.

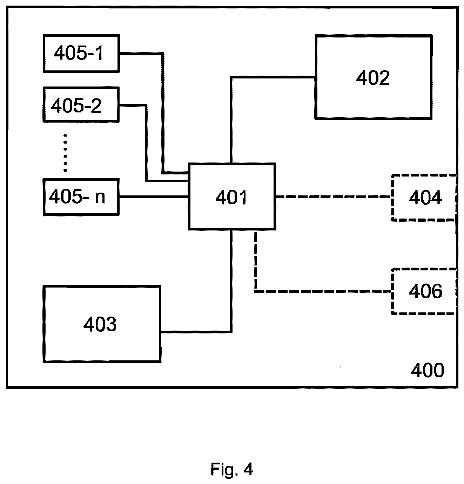

Die Figur 4 zeigt einen beispielhaften schematischen Aufbau eines medizintechnischen Geräts 400 gemäß der vorliegenden Lehre.

Das medizintechnische Gerät 400 umfasst zumindest ein Steuergerät 401. Oftmals verfügen medizintechnische Geräte über mehrere Steuergeräte, oftmals als Prozessoren oder Mikrokontroller ausgeführt. Es ist für die vorliegende Lehre unerheblich, ob die beschriebenen Verfahren von einem einzigen Steuergerät oder von einer Vielzahl von Steuergeräten ausgeführt werden.

Das Steuergerät 401 ist datentechnisch mit anderen Komponenten verbunden. Über diese Verbindungen werden Daten ausgetauscht, beispielsweise Steuerbefehle oder Parameterwerte.

Weiterhin verfügt das medizintechnische Gerät 400 über eine Bedienerschnittstelle 402, über die Informationen ein- und ausgebbar sind. Eine solche Bedienerschnittstelle 402 ist in einer Ausführungsform ein Touchscreen-Display.

Es ist für die Bedienerschnittstelle 402 unerheblich, ob die Ein- und Ausgabemöglichkeit in einem einzigen Gerät kombiniert sind, wie beispielsweise in einem Touchscreen-Display, oder ob die Ein- und Ausgabe von Informationen über separate Vorrichtungen erfolgen, beispielsweise durch einen konventionellen Bildschirm in Verbindung mit einer separaten berührempfindlichen Fläche, einem sogenannten Touchpad. Im Lichte der Offenbarung der vorliegenden Erfindung kann eine Bedienerschnittstelle auch getrennte Ein- und Ausgabevorrichtungen umfassen.

Weiterhin umfasst das medizintechnische Gerät 400 ein oder mehrere Betriebsmittel 405-1, 405-2 bis 405-n, wie beispielsweise eine Pumpe, ein Ventil oder einen Sensor.

Eine mittelbare Verbindung eines Betriebsmittels 405-x mit dem Steuergerät 401 bedeutet, dass eine datentechnische Verbindung nicht unmittelbar vorhanden ist, sondern beispielsweise weitere Vorrichtungen zur Aufbereitung der Daten zwischengeschaltet sind. Beispielsweise können analoge Signale von Sensoren zuvor durch einen zwischengeschalteten analog/digital-Konverter in digitale Daten gewandelt werden, die dann dem Steuergerät zugeführt werden.

Das medizintechnische Gerät 400 verfügt über internen Speicher 403, in dem Software zur Programmierung des Steuergeräts 401 gespeichert werden kann. Darüber hinaus können beliebige Daten, wie beispielsweise das medizintechnische Gerät betreffende Dokumente in digitaler Form als Dateien im Speicher 403 abgespeichert sein.

Weiterhin kann das medizintechnische Gerät 400 eine Netzwerkschnittstelle 404 umfassen. Über diese Netzwerkschnittstelle kann das Steuergerät 401 über eine Netzwerkverbindung auf weitere Geräte, wie beispielsweise einen Zentralrechner oder einen Cloudspeicher, zugreifen, um in schon beschriebener Weise Zugriff auf eine oder mehrere das medizintechnische Gerät betreffende Dateien zu erlangen.

Über die Netzwerkschnittstelle kann aber auch von außen Zugriff auf den internen Speicher erlangt werden, um beispielsweise neue Software einzuspielen, oder um die optional dort vorgehaltenen eine oder mehrere, das medizintechnische Gerät betreffenden Dateien abzuspeichern oder zu aktualisieren.

Die Netzwerkschnittstelle kann kabelgebunden, bspw. nach dem RJ45 Standard, oder kabellos (WLAN, Bluetooth, Infrarot etc.) ausgeführt sein.

Das medizintechnische Gerät kann weiterhin eine Autorisierungsvorrichtung 406 umfassen, über die die Autorisierung des Bedieners eingebbar ist. Eine solche Autorisierungsvorrichtung ist beispielsweise ein Kartenlesegerät, das Speicherkarten (Magnetspeicherkarten, SmartCards etc.) einlesen kann, wobei die auf der Speicherkarte gespeicherte Information für die Identität des Besitzers der Speicherkarte charakteristisch ist. Weiter Autorisierungsvorrichtungen sind beispielsweise Irisscanner, Fingerabdruckscanner, RFID Lesegeräte etc.

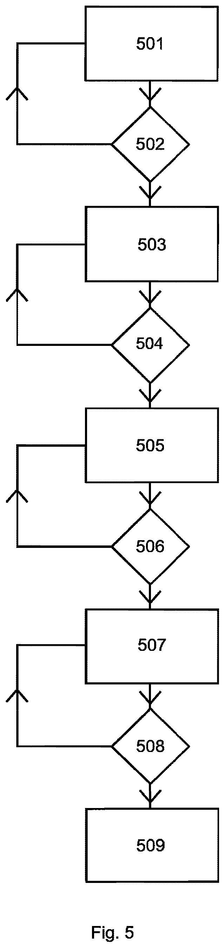

Die Figur 5 zeigt ein Ablaufdiagramm eines Verfahrens im Einklang mit der vorliegenden Lehre.

Im Schritt 501 werden auf der grafischen Bedienerschnittstelle Informationen ausgegeben, die das medizintechnische Gerät betreffen und Eingabemöglichkeiten für einstellbare Betriebsparameter angezeigt. Figur 2 oben zeigt eine entsprechende Bildschirmansicht.

In der Regel sind nicht alle einstellbaren und/oder ausgebbaren Informationen gleichzeitig auf einer Bildschirmansicht ausgebbar und/oder eingebbar. Unter einer Bildschirmansicht wird hier die Gesamtheit der Bildschirmanzeige zu einem beliebigen Zeitpunkt verstanden. Ändert sich die Bildschirmanzeige, wird folglich eine andere Seite angezeigt. Da es oftmals eine große Anzahl von Informationen und einstellbaren Betriebsparamater bei medizintechnischen Geräten gibt, werden diese nicht alle gleichzeitig auf einer Bildschirmansicht angezeigt, um die Übersichtlichkeit zu erhöhen. Information und Eingabemöglichkeiten für einstellbaren Betriebsparameter werden hierbei oftmals thematisch zusammengefasst und innerhalb eigener Menus angezeigt.

Weiterhin umfasst der Schritt 501, die Auswahl einer Suchfunktion zu ermöglichen, mittels derer Textstrings eingebbar sind. Die Auswahl einer Suchfunktion wird beispielsweise durch Vorhalten eines entsprechenden Touch-Keys (203) auf allen, oder einer Vielzahl von Menu-Ansichten ermöglicht.

Im Schritt 502 wird geprüft, ob die Suchfunktion ausgewählt wird, beispielsweise durch Berühren des entsprechenden Touch-Keys (203). Ist das der Fall, erfolgt Schritt 503. Erfolgt keine Auswahl der Suchfunktion, wird der Schritt 501 weiter ausgeführt.

Im Schritt 503 wird die Suchfunktion aktiviert. Dies kann beispielsweise durch Öffnen des Fensters 204 geschehen.

Im Schritt 504 erfolgt die Überprüfung einer Eingabe in die Suchfunktion. Wird keine Eingabe detektiert, wird der Schritt 503 weiter ausgeführt. Wird eine Eingabe detektiert, erfolgt im Schritt 505 die Suche nach dem eingegebenen Textstring oder Teilen hiervon, oder nach Textstrings, die hinsichtlich ihrer Schreibweise oder hinsichtlich ihrer Aussprache zumindest teilweise mit dem eingegebenen Begriff oder Teilen hiervon übereinstimmen. Diese Suche erfolgt innerhalb von einer oder mehreren Dateien, die dem medizintechnischen Gerät zugänglich sind. Der Speicherort dieser Dateien spielt hierbei keine Rolle, wesentlich ist, dass das medizintechnische Gerät datentechnisch auf diese Dateien Zugriff hat. Ebenfalls im Schritt 505 erfolgt die Anzeige der gefundenen Textstrings, die auswählbar sind. In der unteren Bildschirmansicht der Figur 2 ist die Anzeige der gefundenen Begriffe mit dem Bezugszeichen 206 gekennzeichnet.

Im Schritt 506 erfolgt die Erkennung einer Auswahl eines bestimmten Textstrings aus den durch den Suchvorgang gefundenen Textstrings. Wird keine Auswahl detektiert, wird der Schritt 505 weiter ausgeführt. Wird eine Auswahl detektiert, erfolgt im Schritt 507 die Anzeige des ausgewählten Ausschnitts der einen oder mehrerer Dateien, die den ausgewählten Textstring beinhalten (Figur 2, Bezugszeichen 210 und 211). Teile des angezeigten Ausschnitts, die Betriebsmittel betreffen, können auswählbar sein. Dies ist beispielsweise durch die Unterstreichung des entsprechenden Teils (beispielsweise "Heparinmenu", Fig. 2 unten) gekennzeichnet.

Im Schritt 508 wird geprüft, ob ein auswählbarer Teil innerhalb des angezeigten Ausschnitts der einen oder mehrerer Dateien ausgewählt wird. Wird keine Auswahl detektiert, wird der Schritt 507 weiter ausgeführt. Wird eine solche Auswahl detektiert, erfolgt im Schritt 509 die Anzeige von Betriebsparametern des betreffenden Betriebsmittels und/oder von zumindest einer von dem betreffenden Betriebsmittel erzeugten und auslesbaren Ausgangsgröße auf der grafischen Bedienerschnittstelle. Es kann auch eine Eingabemöglichkeit für einstellbare Betriebsparameter des betreffenden Betriebsmittels auf der grafischen Bedienerschnittstelle angezeigt werden (Fig. 3, Bezugszeichen 301).

Durch die vorliegende Erfindung wird die Bedienung von medizintechnischen Geräten vereinfacht und sicherer gestaltet. Sie gibt vor allem dem unerfahrenen Bediener die Möglichkeit, Informationen innerhalb der Struktur einer grafischen Bedieneroberfläche schnell zu finden, sowie Parameter von Betriebsmitteln des medizintechnischen Geräts schnell einzusehen oder einzugeben. Der Betrieb des medizintechnischen Geräts wird somit vereinfacht und die Betriebssicherheit erhöht.

## Patentansprüche

1. Medizintechnisches Gerät umfassend zumindest eine Eingabevorrichtung und zumindest eine Ausgabevorrichtung, wobei die zumindest eine Eingabevorrichtung zur Eingabe von das medizintechnische Gerät betreffenden Informationen eingerichtet ist, und wobei die zumindest eine Ausgabevorrichtung zur Ausgabe von das medizintechnische Gerät betreffenden Informationen eingerichtet ist, und zumindest ein Betriebsmittel, wobei das zumindest eine Betriebsmittel zumindest einen einstellbaren und/oder auslesbaren Betriebsparameter und/oder zumindest eine von dem Betriebsmittel erzeugte und auslesbare Ausgangsgröße aufweist, und ein Steuergerät, welches datentechnisch zumindest mittelbar mit dem zumindest einen Betriebsmittel und der zumindest einen Eingabevorrichtung und der zumindest einen Ausgabevorrichtung verbunden ist, wobei das Steuergerät dazu konfiguriert ist, Informationen, die das medizintechnische Gerät betreffen, auszugeben und Eingabemöglichkeiten für die einstellbaren Betriebsparameter anzuzeigen,
und wobei das Steuergerät datentechnischen Zugriff auf zumindest eine das medizintechnische Gerät betreffende Datei hat, und so konfiguriert ist, dass Textteile oder Grafiken der zumindest einen Datei, die ein Betriebsmittel betreffen, auswählbar sind, und dass nach der Auswahl eines solchen Textteils oder einer solchen Grafik zumindest ein Betriebsparameter des Betriebsmittels ausgelesen und ausgegeben wird und/oder zumindest eine von dem betreffenden Betriebsmittel erzeugte und auslesbare Ausgangsgröße ausgelesen und ausgegeben wird, und dass nach der Auswahl eines solchen Teils die Eingabe eines einstellbaren Betriebsparameters des betreffenden Betriebsmittels über eine angezeigte Eingabemöglichkeit für die einstellbaren Betriebsparameter ermöglicht wird,
und wobei
die Datei ausschließlich textbasierte und/oder grafikbasierte Informationen umfasst und keinen zur Steuerung des medizintechnischen Gerätes verwendbaren ausführbaren Programmcode.

2. Medizintechnisches Gerät nach Anspruch 1, wobei das Steuergerät dazu eingerichtet ist, die Auswahl einer Suchfunktion zu ermöglichen, mittels derer ein Textstring eingebbar ist, und wobei der eingegebene Textstring innerhalb der zumindest einen Datei gesucht
wird, wobei Textteile oder Ausschnitte der Datei, die den gesuchten Textstring beinhalten, auswählbar sind.

3. Medizintechnisches Gerät nach Anspruch 2, wonach auch Nachrichten, die den gesuchten Textstring betreffen, ausgegeben werden.

4. Medizintechnisches Gerät nach einem der vorhergehenden Ansprüche, weiterhin umfassend eine Autorisierungsvorrichtung, welche dazu eingerichtet ist, zumindest eines für die Identität oder für die Autorisierung einer Person charakteristisches Merkmal einlesen zu können , wonach das Steuergerät zusätzlich dazu konfiguriert ist, die Anzahl der auswählbaren Ausschnitte der Datei und/oder die Anzahl der auswählbaren Textteile oder Grafiken eines ausgegebenen Teils der Datei, die ein Betriebsmittel betreffen, abhängig von einer mit der Autorisierungsvorrichtung eingelesenen Identität oder Autorisierung einer Person zu gestalten.

5. Medizintechnisches Gerät nach einem der vorhergehenden Ansprüche mit einer Speichervorrichtung, wobei die das medizintechnische Gerät betreffenden Datei in der Speichervorrichtung gespeichert ist.

6. Medizintechnisches Gerät nach Anspruch 5 umfassend eine Netzwerkschnittstelle, wobei die Speichervorrichtung mittels der Netzwerkschnittstelle beschreibbar ist.

7. Medizintechnisches Gerät nach einem der Ansprüche 1 bis 5 mit einer Netzwerkschnittstelle, wobei die das medizintechnische Gerät betreffende Datei in der Speichervorrichtung eines externen Geräts gespeichert ist und der datentechnische Zugriff auf die das medizintechnische Gerät betreffende Datei mittels der Netzwerkschnittstelle erfolgt.

8. Medizintechnisches Gerät nach einem der vorhergehenden Ansprüche, wobei die zumindest eine Eingabevorrichtung eine Vorrichtung zur Eingabe akustischer Informationen umfasst und/oder die zumindest eine Ausgabevorrichtung eine Vorrichtungen zur Ausgabe von akustischen Informationen umfasst, und die Eingabe zumindest teilweise mittels der Vorrichtung zur Eingabe von akustischen Informationen und/oder die Ausgabe zumindest teilweise mittels der Vorrichtung zur Ausgabe von akustischen Informationen erfolgen.

9. Medizintechnisches Gerät nach einem der vorhergehenden Ansprüche, wobei das medizintechnische Gerät ein Blutbehandlungsgerät ist und insbesondere zur Hämodialyse, Peritonealdiaylse, Hämofiltration, Hämodiafiltration oder Plasmapherese eingerichtet ist.

10. Verfahren zur Informationseingabe und zur Informationsausgabe für ein medizintechnisches Gerät umfassend die Schritte:
Ausgeben von Informationen, die das medizintechnische Gerät betreffen, und
Vorhalten von Eingabemöglichkeiten für einstellbare Betriebsparameter von Betriebsmitteln des medizintechnischen Geräts,
wobei
Textteile oder Grafiken zumindest einer das medizintechnische Gerät betreffenden Datei, die ein Betriebsmittel betreffen, auswählbar sind, und wobei nach der Auswahl eines solchen Textteils oder einer solchen Grafik zumindest ein Betriebsparameter des Betriebsmittels ausgelesen und ausgegeben wird und/oder zumindest eine von dem betreffenden Betriebsmittel erzeugte und auslesbare Ausgangsgröße ausgelesen und ausgegeben wird, und wobei nach der Auswahl eines solchen Teils die Eingabe eines einstellbaren Betriebsparameters des betreffenden Betriebsmittels über eine angezeigte Eingabemöglichkeit für die einstellbaren Betriebsparameter ermöglicht wird.
und wobei die Datei ausschließlich textbasierte und/oder grafikbasierte Informationen umfasst und keinen zur Steuerung des medizintechnischen Gerätes verwendbaren ausführbaren Programmcode.

11. Verfahren nach Anspruch 10, wobei die Auswahl einer Suchfunktion ermöglicht wird, mittels derer ein Textstring eingebbar ist, und wobei der eingegebene Textstring innerhalb der zumindest einen Datei gesucht wird, wobei Textteile oder Ausschnitte der Datei, die den gesuchten Textstring beinhalten, auswählbar sind.

12. Verfahren nach Anspruch 11, wonach auch Nachrichten, die den gesuchten Textstring betreffen, ausgegeben werden.

13. Verfahren nach einem der Ansprüche 10 bis 12, wonach zumindest eines für die Identität oder für die Autorisierung einer Person charakteristische Merkmal eingelesen wird, und wobei die Anzahl der auswählbaren Ausschnitte der Datei und/oder die Anzahl der auswählbaren Textteile oder Grafiken eines ausgegebenen Ausschnitts der Datei, der ein Betriebsmittel betrifft, abhängig von der eingelesenen Identität oder Autorisierung einer Person ist.

14. Verfahren nach einem der Ansprüche 10 bis 13, wobei die Eingabe zumindest teilweise mittels Vorrichtungen zur Eingabe von akustischen Informationen und/oder die Ausgabe zumindest teilweise mittels Vorrichtungen zur Ausgabe von akustischen Informationen erfolgen.

15. Verfahren nach einem der Ansprüche 10 bis 14, wobei das medizintechnische Gerät ein Blutbehandlungsgerät ist und insbesondere zur Hämodialyse, Peritonealdiaylse, Hämofiltration, Hämodiafiltration oder Plasmapherese eingerichtet ist.

## Claims

1. Medical device comprising at least one input apparatus and at least one output apparatus, wherein the at least one input apparatus is configured to input information relating to the medical device and wherein the at least one output apparatus is configured to output information relating to the medical device, and at least one operating means, wherein the at least one operating means has at least one adjustable and/or readable operating parameter and/or at least one output variable which is readable and generated by the operating means, and a controller which is data-connected at least indirectly to the at least one operating means and the at least one input apparatus and the at least one output apparatus, wherein the controller is configured to output information relating to the medical device and display input options for the adjustable operating parameters, and wherein the controller has data access to at least one file relating to the medical device and is configured so that text portions or graphics of the at least one file which relate to an operating means are selectable and that, following the selection of such a text portion or such a graphic, at least one operating parameter of the operating means is read and output and/or at least one output variable readable and generated by the relevant operating means is read and output and that, following the selection of such a portion, the input of an adjustable operating parameter of the relevant operating means is rendered possible by way of a displayed input option for the adjustable operating parameters,
and wherein
the file only comprises text-based and/or graphics-based information and no executable program code usable for controlling the medical device.

2. Medical device according to Claim 1, wherein the controller is configured to enable the selection of a search function, by means of which a text string is able to be entered, and wherein the entered text string is searched within the at least one file, wherein text portions or portions of the file which contain the searched text string are selectable.

3. Medical device according to Claim 2, according to which messages relating to the searched text string are also output.

4. Medical device according to any of the preceding claims, also comprising an authorization apparatus configured to be able to read at least one feature which is characteristic for the identity or for the authorization of a person, according to which the controller is additionally configured to set the number of selectable portions of the file and/or the number of the selectable text portion or graphics of an output part of the file which relate to an operating means in a manner dependent on an identity or authorization of a person read by way of the authorization apparatus.

5. Medical device according to any of the preceding claims, having a storage apparatus, wherein the file relating to the medical device is stored in the storage apparatus.

6. Medical device according to Claim 5, comprising a network interface, wherein the storage device is writable by means of the network interface.

7. Medical device according to any of Claims 1 to 5, having a network interface, wherein the file relating to the medical device is stored in the storage apparatus of an external device and the data access to the file relating to the medical device is implemented by means of the network interface.

8. Medical device according to any of the preceding claims, wherein the at least one input apparatus comprises an apparatus for the input of acoustic information and/or the at least one output apparatus comprises an apparatus for the output of acoustic information, and the input is at least partly implemented by means of the apparatus for the input of acoustic information and/or the output is at least partly implemented by means of the apparatus for the output of acoustic information.

9. Medical device according to any of the preceding claims, wherein the medical device is a blood treatment device and in particular configured for haemodialysis, peritoneal dialysis, haemofiltration, haemodiafiltration or plasmapheresis.

10. Method of information input and information output for a medical device, comprising the steps of:
outputting information relating to the medical device, and
keeping available input options for adjustable operating parameters of operating means of the medical device,
wherein
text portions or graphics which are in at least one file relating to the medical device and which relate to an operating means are selectable and wherein, following the selection of such a text portion or such graphics, at least one operating parameter of the operating means is read and output and/or at least one output variable which is readable and generated by the relevant operating means is read and output, and wherein, following the selection of such a portion, the input of an adjustable operating parameter of the relevant operating means is enabled by way of a displayed input option for the adjustable operating parameters,
and wherein the file only comprises text-based and/or graphics-based information and no executable program code usable for controlling the medical device.

11. Method according to Claim 10, wherein the selection of a search function is made possible, by means of which a text string is able to be entered, and wherein the entered text string is searched within the at least one file, wherein text portions or portions of the file which contain the searched text string are selectable.

12. Method according to Claim 11, according to which messages relating to the searched text string are also output.

13. Method according to any of Claims 10 to 12, according to which at least one feature which is characteristic for the identity or for the authorization of a person is read, and wherein the number of selectable portions of the file and/or the number of selectable text portions or graphics of an output portion of the file relating to an operating means is dependent on the read identity or authorization of a person.

14. Method according to any of Claims 10 to 13, wherein the input is at least partly implemented by means of apparatuses for the input of acoustic information and/or the output is at least partly implemented by means of apparatuses for the output of acoustic information.

15. Method according to any of Claims 10 to 14, wherein the medical device is a blood treatment device and in particular configured for haemodialysis, peritoneal dialysis, haemofiltration, haemodiafiltration or plasmapheresis.

## Revendications

1. Appareil médical comprenant au moins un dispositif d'entrée et au moins un dispositif de sortie, l'au moins un dispositif d'entrée étant conçu pour entrer des informations relatives à l'appareil médical, et l'au moins un dispositif de sortie étant conçu pour délivrer des informations relatives à l'appareil médical, et au moins un moyen de fonctionnement, l'au moins un moyen de fonctionnement comportant au moins un paramètre de fonctionnement réglable et/ou lisible et/ou au moins une grandeur de sortie générée et lisible par le moyen de fonctionnement, et une unité de commande qui est relié du point de vue informatique au moins indirectement à l'au moins un moyen de fonctionnement et à l'au moins un dispositif d'entrée et à l'au moins un dispositif de sortie, l'unité de commande étant conçue pour délivrer des informations relatives à l'appareil médical et pour afficher des options d'entrée pour les paramètres de fonctionnement réglables,
et
l'unité de commande ayant un accès informatique à au moins un fichier relatif à l'appareil médical et étant conçue de manière à ce que des parties de texte ou des graphiques de l'au moins un fichier relatif à un moyen de fonctionnement puissent être sélectionnés, et à ce que, après la sélection d'une telle partie de texte ou d'un tel graphique, au moins un paramètre de fonctionnement du moyen de fonctionnement soit lu et délivré et/ou au moins une grandeur de sortie générée et lisible par ledit moyen de fonctionnement soit lue et délivrée, et à ce que, après la sélection d'une telle une partie, permettre l'entrée d'un paramètre de fonctionnement réglable dudit moyen de fonctionnement par le biais d'une option d'entrée affichée du paramètre de fonctionnement réglable, et
le fichier ne comprenant que des informations textuelles et/ou graphiques et aucun code de programme exécutable pouvant être utilisé pour commander l'appareil médical.

2. Appareil médical selon la revendication 1, le dispositif de commande étant conçu pour permettre la sélection d'une fonction de recherche permettant d'entrée une chaîne de texte, et la chaîne de texte entrée étant recherchée dans au moins un fichier, les parties de texte ou extraits du fichier qui contiennent la chaîne de texte recherchée pouvant être sélectionnés.

3. Appareil médical selon la revendication 2, des messages relatifs à la chaîne de texte recherchée étant délivrés.

4. Appareil médical selon l'une des revendications précédentes, comprenant en outre un dispositif d'autorisation qui est conçu pour pouvoir lire au moins une caractéristique relative à l'identité ou à l'autorisation d'une personne, puis l'unité de commande étant conçue en outre pour établir le nombre d'extraits sélectionnables du fichier et/ou le nombre de parties de texte ou de graphiques sélectionnables d'une partie délivrée du fichier qui concernent un moyen de fonctionnement, en fonction d'une identité ou autorisation d'une personne lue avec le dispositif d'autorisation.

5. Appareil médical selon l'une des revendications précédentes comprenant un dispositif de mémorisation, le fichier relatif à l'appareil médical étant mémorisé dans le dispositif de mémorisation.

6. Appareil médical selon la revendication 5 comprenant une interface réseau, l'écriture dans le dispositif de mémorisation pouvant être effectuée par le biais de l'interface réseau.

7. Appareil médical selon l'une des revendications 1 à 5 comprenant une interface réseau, le fichier relatif à l'appareil médical étant mémorisé dans le dispositif de mémorisation d'une unité externe et l'accès informatique à l'appareil médical étant effectué au moyen de l'interface réseau.

8. Appareil médical selon l'une des revendications précédentes, l'au moins un dispositif d'entrée comprenant un dispositif d'entrée d'informations acoustiques et/ou l'au moins un dispositif de sortie comprenant un dispositif de sortie d'informations acoustiques, et l'entrée étant effectuée au moins partiellement au moyen du dispositif d'entrée d'informations acoustiques et/ou la sortie étant effectuée au moins partiellement au moyen du dispositif de sortie d'informations acoustiques.

9. Appareil médical selon l'une des revendications précédentes, l'appareil médical étant un appareil de traitement du sang et étant conçu notamment pour l'hémodialyse, la dialyse péritonéale, l'hémofiltration, l'hémodiafiltration ou la plasmaphérèse.

10. Procédé d'entrée d'informations et de sortie d'informations destiné à un appareil médical, ledit procédé comprenant les étapes suivantes :
délivrer des informations relatives à l'appareil médical et
présenter des options d'entrée de paramètres de fonctionnement réglables de moyens de fonctionnement de l'appareil médical,
des parties de texte ou des graphiques d'au moins un fichier relatif à l'appareil médical, lesquels concernent un moyen de fonctionnement, pouvant être sélectionnés et, après la sélection d'une telle partie de texte ou d'un tel graphique, au moins un paramètre de fonctionnement du moyen de fonctionnement étant lu et délivré et/ou au moins une grandeur de sortie générée et lisible par ledit moyen de fonctionnement étant lue et délivrée et, après la sélection d'une telle partie, l'entrée d'un paramètre de fonctionnement réglable dudit moyen de fonctionnement étant rendue possible par le biais d'une option d'entrée affichée pour les paramètres de fonctionnement réglables, et
le fichier ne comprenant que des informations textuelles et/ou graphiques et aucun code de programme exécutable pouvant être utilisé pour commander l'appareil médical.

11. Procédé selon la revendication 10, la sélection d'une fonction de recherche étant rendue possible qui permet d'entrer une chaîne de texte, et la chaîne de texte entrée étant recherchée dans au moins un fichier, les parties de texte ou extraits du fichier qui contiennent la chaîne de texte recherchée pouvant être sélectionnées.

12. Procédé selon la revendication 11, des messages relatifs à la chaîne de texte recherchée étant également délivrés.

13. Procédé selon l'une des revendications 10 à 12, au moins une caractéristique relative à l'identité ou l'autorisation d'une personne étant lue, et le nombre d'extraits sélectionnables du fichier et/ou le nombre de parties de texte ou de graphiques sélectionnables d'un extrait délivré du fichier qui concerne un moyen de fonctionnement dépendant de l'identité ou de l'autorisation d'une personne qui ont été lues.

14. Procédé selon l'une des revendications 10 à 13, l'entrée étant effectuée au moins partiellement au moyen de dispositifs d'entrée d'informations acoustiques et/ou la sortie étant effectuée au moins partiellement au moyen de dispositifs de délivrance d'informations acoustiques.

15. Procédé selon l'une des revendications 10 à 14, l'appareil médical étant un dispositif de traitement du sang et étant conçu notamment pour l'hémodialyse, la dialyse péritonéale, l'hémofiltration, l'hémodiafiltration ou la plasmaphérèse.
